# FASCICULE DE BREVET EUROPEEN

(11) **EP 3 405 715 B1**
(45) Date de publication et mention de la délivrance du brevet: **19.02.2020**
(21) Numéro de dépôt: 17706540.6
(22) Date de dépôt: 18.01.2017
(51) Int. Cl.: F16M 11/20, F16M 11/24, F16M 13/02, A61G 12/00, A61B 90/50

(54) **DISPOSITIF DE SUSPENSION MEDICAL A BRAS DE DEPORT**
MEDIZINISCHE SUSPENSIONSVORRICHTUNG MIT EINEM VERSETZTEN ARM
MEDICAL SUSPENSION DEVICE COMPRISING AN OFFSET ARM

(30) Priorité: 19.01.2016 FR 1650394
(43) Date de publication de la demande: 28.11.2018
(73) Titulaire: Maquet SAS, 45160 Ardon (FR)
(72) Inventeur: RAVALITERA, Pierre, 45160 Ardon (FR); GUILLEMINOT, Bertrand, 45160 Ardon (FR); SENELIER, Gregory, 45160 Ardon (FR)
(74) Mandataire: Prugneau, Philippe
(86) Numéro de dépôt international: PCT/FR2017/050102
(87) Numéro de publication internationale: WO 2017/125677

(56) Documents cités:
- EP-A1- 1 239 805
- FR-A1- 2 974 473
- FR-A3- 2 662 779

## Description

### Domaine technique

Le domaine de l'invention concerne les dispositifs de suspension permettant de suspendre des équipements sous une structure support, comprenant une platine d'ancrage destinée à être ancrée axialement à la structure support, deux bras de distribution réglables en débattement destinés à porter les équipements, chaque bras de distribution comprenant un bras de suspension ayant une extrémité articulée à la platine d'ancrage par l'intermédiaire d'une articulation pivot permettant une rotation du bras de suspension dans un plan horizontal et un bras ressort articulé à l'autre extrémité du bras de suspension pour être inclinable verticalement par rapport au bras de suspension, l'autre extrémité du bras ressort portant un équipement.

### Technique antérieure

En milieu médical, notamment dans une salle opératoire, des équipements médicaux tels que des dispositifs d'éclairage, des dispositifs de surveillance ou de contrôle avec écrans et caméras, des fluides médicaux sont généralement suspendus à une structure support via un dispositif de suspension accroché à une platine d'ancrage ancrée à la structure support, tel qu'un plafond. Le dispositif de suspension permet de répartir les multiples équipements par des bras de distribution réglables autour d'une zone d'utilisation, le plus souvent une table d'opération centrée dans la salle opératoire. Un bras de distribution comprend généralement un bras de suspension relié d'un coté à la platine d'ancrage et de l'autre à un bras ressort qui porte un ou plusieurs équipements médicaux.

On connaît du document FR 2662779 une suspension plafonnière pour la fixation d'un téliviseur ou tout autre appareil de forme similaire. On connait en outre des documents EP 1239805, EP 1442246, EP1478876 des dispositifs de suspension médicaux supportant un ou plusieurs équipements avec des bras de distribution articulés. Ces documents décrivent en outre des dispositifs de suspension accrochés à une platine d'ancrage positionnée le plus souvent au centre de la salle opératoire, de telle sorte que la table opératoire est alignée avec la platine d'ancrage.

Il est reconnu par le personnel médical qu'au cours d'une opération le déplacement des bras articulés portant les multiples équipements peut induire des collisions entre des équipements fixes ou mobiles. Les collisions peuvent être du type le bras ressort entrant en contact avec son bras de suspension, ou encore une coupole d'éclairage du dispositif d'éclairage fixé à un bras articulé cognant un bras de suspension ou un bras ressort d'un autre bras articulé.

De plus même si le personnel médical est souvent satisfait de la fonction primaire des dispositifs de suspension, il se plaint souvent des difficultés de positionnement avec des efforts importants à fournir pour la manipulation des dispositifs de suspension, et il mentionne aussi une dérive possible des différents équipements. Un autre grief du personnel médical concerne des positionnements d'équipements, tels que des dispositifs d'éclairage, impossibles à atteindre sur la zone d'utilisation, créant ainsi des singularités sur la zone d'utilisation.

Afin de répondre en partie à ces inconvénients, des fabricants de dispositifs de suspension d'équipements médicaux dans une salle opératoire proposent d'ancrer des dispositifs de suspension sur les cotés de la structure support de la salle opératoire, de sorte que des ancrages soient en position 9-3 ou 12-6 (ces chiffres correspondant au positionnement des aiguilles d'une montre) par rapport à la table opératoire positionnée au centre de la salle opératoire. Les installations avec des ancrages de type 9-3 semblent apporter une meilleure utilisation de l'espace tout en étant compatible avec les flux laminaires qui sont prévus initialement pour des ancrages centralisés.

Néanmoins, les salles opératoires ne sont pas toujours compatibles pour de telles installations qui nécessitent plusieurs points d'ancrage, les salles conventionnelles permettant uniquement un ancrage centralisé de dispositif de suspension.

Le document FR 2974473 montre un dispositif de suspension selon le préambule de la revendication 1.

### Exposé de l'invention

Le but de l'invention est donc de remédier à ces inconvénients en proposant un dispositif de suspension présentant les avantages d'un agencement 9-3 des dispositifs de suspension compatible avec un ancrage centralisé d'une salle opératoire.

A cet effet, l'invention a pour objet un dispositif de suspension permettant de suspendre des équipements sous une structure support, comprenant une platine d'ancrage destinée à être ancrée axialement à la structure support, deux bras de distribution réglables en débattement destinés à porter les équipements , chaque bras de distribution comprenant un bras de suspension ayant une extrémité articulée à la platine d'ancrage par l'intermédiaire d'une articulation pivot permettant une rotation du bras de suspension dans un plan horizontal et un bras ressort articulé à l'autre extrémité du bras de suspension pour être inclinable verticalement par rapport au bras de suspension, l'autre extrémité du bras ressort portant un équipement, caractérisé en ce que chaque articulation pivot d'un bras de suspension est disposée à l'extrémité d'un bras de déport pour être déportée horizontalement d'une certaine distance de déport par rapport à l'axe de la platine d'ancrage qui est supérieure à la longueur du bras de suspension et en ce qu'il comprend en outre un moyen de liaison qui solidarise de manière fixe le bras de déport à l'axe de la platine d'ancrage de telle manière qu'il reste fixe par rapport à la platine d'ancrage pendant un pivotement du bras de distribution dans le plan horizontal.

Le dispositif de suspension selon l'invention peut ainsi avantageusement présenter les particularités suivantes :
- il comprend deux bras de déport qui sont en alignement mutuel de part et d'autre d'un point central de la platine d'ancrage ;
- l'équipement est un équipement médical.

Avec cet agencement, il est possible de créer un déport artificiel des équipements portés par les bras de distributions (le bras de suspension et le bras ressort) à partir d'un ancrage centralisé afin d'obtenir une configuration de type 9-3 connue pour limiter les collisions et le nombre de position singulière dans la zone d'utilisation, tout en étant compatible avec les flux laminaires d'une salle opératoire à ancrage centralisé.

Ce déport artificiel est une solution économique et rapide de modification de l'agencement des équipements dans la salle opératoire autour de la zone d'utilisation car il est compatible avec une installation centralisée existante, il permet de réduire le nombre d'ancrage et de diminuer le nombre d'articulation tournante et/ou pivotante par rapport aux dispositifs de suspensions de l'état de l'art.

### Description sommaire des dessins

La présente invention sera mieux comprise et d'autres avantages apparaîtront à la lecture de la description détaillée de plusieurs modes de réalisation pris à titre d'exemples nullement limitatifs et illustrés par les dessins annexés dans lesquels :
- la figure 1 est une représentation schématique d'un dispositif de suspension selon l'art antérieur dans une salle opératoire ;
- la figure 2 est une représentation schématique d'un dispositif de suspension selon l'invention dans une salle opératoire ;
- les figures 3A et 3B est une représentation schématique d'une première et d'une seconde configuration du dispositif de suspension selon l'invention ;
- la figure 4 est une vue en perspective schématique du dispositif de suspension selon l'invention dans une salle opératoire.
- la figure 5 est une vue en perspective schématique du dispositif de suspension selon un autre mode de réalisation de l'invention dans une salle opératoire.

### Description d'un exemple de réalisation

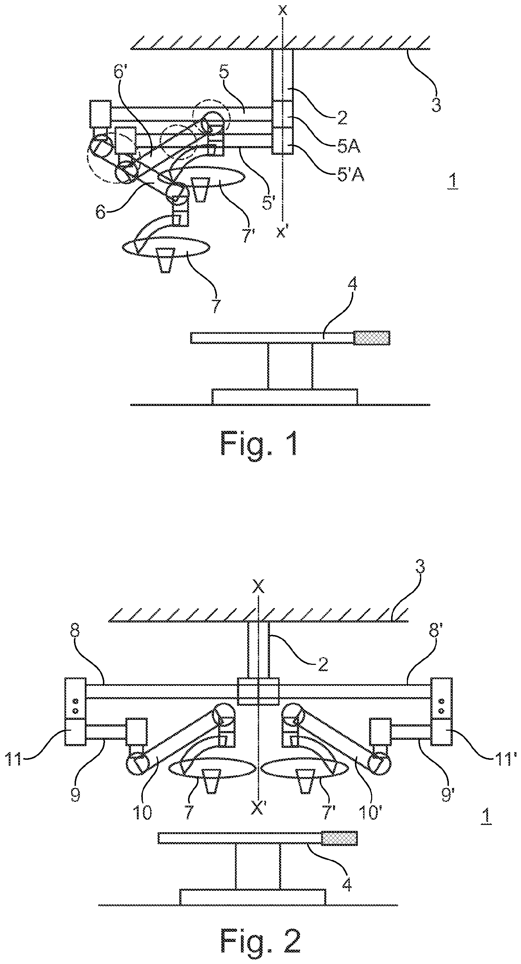

En référence à la figure 1, on a représenté un dispositif de suspension selon l'art antérieur ici dans une salle opératoire 1 avec une platine d'ancrage 2 ancrée à une structure support 3 ou plafond de cette salle opératoire au-dessus d'une table d'opération 4.

Au cours d'une opération médicale, le personnel médical a l'habitude d'aligner à l'aplomb le centre de la table d'opération 4 avec la platine d'ancrage 2.

Sur la figure 1, on a illustré deux bras de distribution réglables articulés chacun par rapport à la platine d'ancrage 2, chacun des bras de distribution portant à leur extrémité libre un équipement 7, 7' médical, ici un éclairage médical.

A la place d'un éclairage médical 7, 7', on pourrait avoir tout autre système électronique, par exemple de surveillance et de contrôle incluant un écran, une caméra. On pourrait avoir comme équipement, un système de distribution d'un fluide, comme un système de perfusion, etc.

Classiquement, chaque bras de distribution comprend un bras de suspension 5, 5' relié d'un coté à la platine d'ancrage 2 par une articulation pivot 5A, 5'A et de l'autre à un bras ressort 6, 6' portant ici sur son extrémité libre une coupole d'éclairage 7, 7' destinée à former une tache d'éclairement sur une zone d'un champ opératoire, ici sur la table d'opération 4.

On a représenté par l'axe vertical XX' l'axe central de la platine d'ancrage 2 qui est aussi l'axe de l'articulation pivot 5A, 5'A à l'extrémité des bras de suspension 5, 5'.

Comme indiqué plus haut, avec ce type de dispositif de suspension, des collisions peuvent avoir lieu entre les deux bras ressorts 6, 6' des deux bras de distribution ou entre un bras ressort 6, 6' et un bras de suspension 5, 5' du même bras de distribution, ou de l'autre bras de distribution.

On a matérialisé par des cercles en pointillés sur la figure 1 les zones de collision où les collisions sont les plus fréquentes.

A partir de la figure 1, on comprend par exemple que le bras ressort 6' interfère avec le bras de suspension 5' du même bras de distribution du fait que le point le plus haut du bras ressort 6' est plus haut que le point le plus bas du bras de suspension 5'. La coupole d'éclairage 7' peut aussi entrer en collision avec le bras de suspension 5'.

Sur la figure 2, on a illustré le dispositif de suspension selon l'invention en place dans la salle opératoire 1 avec la table d'opération 4 alignée verticalement avec la platine d'ancrage 2 suivant son axe central XX'.

Dans le dispositif de suspension selon l'invention, une articulation pivot 11, 11' d'un bras de distribution est déportée de la platine d'ancrage 2 (de son axe central XX') par l'intermédiaire d'un bras de déport 8, 8' fixe qui est interposé entre la platine d'ancrage 2 et l'articulation en question. Une première extrémité du bras de déport 8, 8' est liée à la platine d'ancrage 2 tandis que l'autre extrémité est liée au bras de suspension 9, 9' via l'articulation pivot 11, 11'.

Sur cette figure 2, on a représenté deux bras de déport 8, 8' en alignement mutuel de part et d'autre d'un point central de la platine d'ancrage 2.

Au cours de l'opération médicale, chaque bras de déport tel que 8 ou 8' est maintenu fixe par rapport à la platine d'ancrage 2 pendant un pivotement du bras de distribution.

Comme représenté sur la figure 2, chaque bras de distribution comporte un bras de suspension 9, 9' relié à la platine d'ancrage 2 / bras de déport 8, 8' par l'articulation pivot 11, 11' et un bras ressort 10, 10' pivotant à une extrémité d'un bras de suspension 9, 9' et ayant une autre extrémité libre qui porte un équipement, ici sur la figure 2, une coupole d'éclairage 7, 7' médical.

La liaison fixe entre un bras de déport 8, 8' et la platine d'ancrage 2 peut être du type fourreau/collier avec possibilité de démontage et de réglage en hauteur avant et après une opération médicale. Cette liaison fixe maintient le bras de déport 8, 8' en position fixe par rapport à la platine d'ancrage 2 pendant un mouvement en déplacement du bras de distribution.

Un tel bras de déport 8, 8' peut parfaitement être installé sur une platine d'ancrage 2 existante, en lieu et place d'un bras de suspension.

A la place d'avoir deux bras de déport 8,8' en alignement mutuel, ces deux bras de déport 8, 8' pourraient être disposés en V avec un angle fixe. Dans les deux cas, ils pourraient aussi être superposés l'un par rapport à l'autre.

L'invention s'étend à des configurations d'un dispositif de suspension avec plus de deux bras de distribution à articulation pivot 11, 11' déportée de la platine d'ancrage 2.

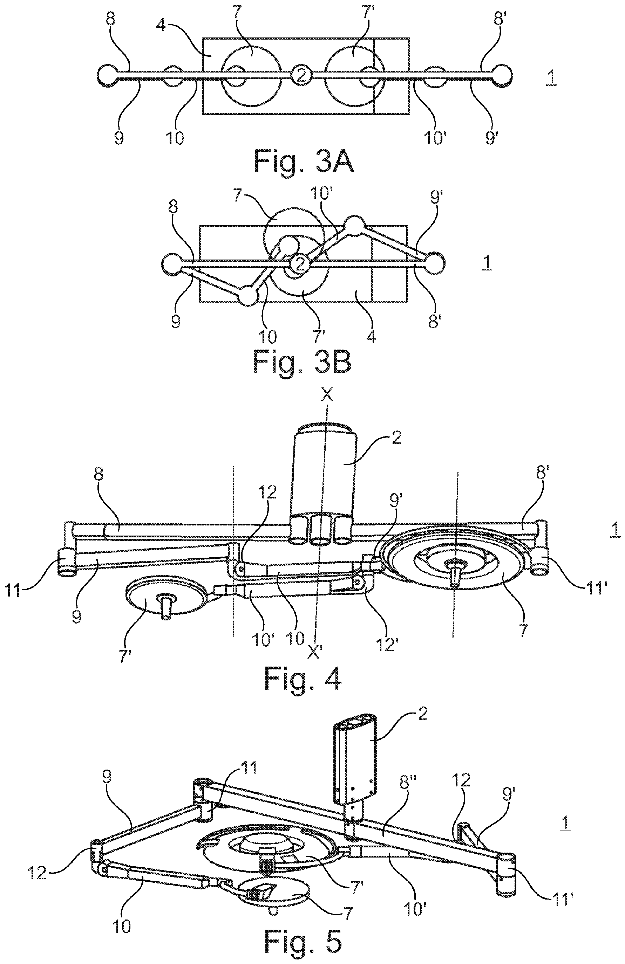

Sur la figure 3A, les deux bras de suspension 9, 9' avec les deux bras ressort 10, 10' sont déployés suivant une configuration 9-3 sous les bras de déport 8,8'.

Les deux coupoles d'éclairage 7, 7' se font face sous les bras de déport 8,8'.

On voit sur cette figure, mais aussi sur la figure 2, que chaque bras de suspension 9, 9' est plus court qu'un bras de déport 8, 8'.

Dans ce cas, il n'y a pas de risque de collision entre les deux bras de suspension 9, 9' mais en plus on peut déplacer la coupole d'éclairage 7, 7' sur une zone plus vaste qu'avec un dispositif classique de suspension en laissant moins de zones de singularités sur le champ opératoire.

Par ailleurs, chaque bras de distribution (bras de suspension 9, 9' avec bras ressort 10, 10') est plus court qu'un bras de déport 8, 8' ce qui fait que les bras ressort 10, 10' ne peuvent pas entrer mutuellement en collision car chaque bras ressort 10, 10' avec une coupole d'éclairage 7, 7' a sa propre zone de mouvement au dessus du champ opératoire.

Il n'y a donc aucun risque de collision entre un bras ressort 10, 10' et un bras de suspension 9, 9' ou entre les coupoles d'éclairage 7, 7' entre elles.

Sur la figure 3B, on a un dispositif de suspension selon l'invention à double bras de distribution, avec deux bras de déport 8, 8' en alignement mutuel, chaque bras de déport 8, 8' étant plus long qu'un bras de suspension 9, 9'. Toutefois, comme visible sur cette figure 3B, les bras ressorts 10,10' se croisent en se faisant face.

Avec cet agencement, chaque coupole d'éclairage 7, 7' peut être positionnée des deux cotés de la table opératoire 4, soit aux pieds soit à la tête d'un patient allongé sur la table.

La figure 4 montre une vue schématique en perspective du dispositif de suspension selon l'invention à double bras de déport 8, 8'.

Comme déjà indiqué ci-dessus, chaque bras de déport 8, 8' est connecté fixement à la platine d'ancrage 2. La connexion peut être démontable ou réglable, par exemple en hauteur suivant l'axe XX'. Sur la figure 4, on a illustré deux connexions décalées du centre de la platine 2 du type fourreau/collier.

Chaque articulation pivot 11, 11' qui relie un bras de suspension 9, 9' à un bras de déport 8, 8' peut aussi être du type fourreau/collier ou moyeu avec possibilité de réglage vertical suivant l'axe XX'.

L'articulation 12, 12' entre un bras de suspension 9, 9' et un bras ressort 10, 10' permet d'incliner vers le bas ou vers le haut le bras de suspension 9, 9' et donc d'éloigner ou de rapprocher la coupole 7, 7' de la table d'opération 4. Cette articulation 12, 12' peut aussi être une articulation pivot à axe de rotation horizontale.

De préférence, afin d'éviter les risques de collision entre les deux bras de suspension 9, 9', ces bras de suspension 9, 9' ne doivent pas pouvoir atteindre l'axe central XX' de la platine d'ancrage 2. Ainsi chaque bras de suspension 9, 9' est plus court qu'un bras de déport 8, 8' .

L'équipement est ici une coupole d'éclairage 7, 7' avec un axe d'éclairage agencé au centre de la coupole d'éclairage 7, 7'. Afin de couvrir l'ensemble de la zone d'utilisation du champ opératoire, l'axe d'éclairage de la coupole d'éclairage 7, 7' doit de préférence pouvoir dépasser l'axe XX' de la platine d'ancrage 2 d'environ 800 mm à +/- 300 mm.

Le bras de déport 8, 8' a de préférence une longueur équivalente à au moins la somme de la longueur du bras ressort 10, 10' et de la coupole d'éclairage jusqu'à son axe d'éclairage.

Il est à noter que l'axe XX' de la platine d'ancrage 2 de la figure 4 est libre pour accueillir d'autres équipements à mettre en suspension.

On comprend que selon l'invention, l'ajout d'un bras de déport 8, 8' au dispositif de suspension est un déport artificiel des équipements 7, 7' dans une salle opératoire 1.

Ce déport artificiel des équipements 7, 7' est compatible au système d'ancrage centralisé dans une salle opératoire 1.

Ce déport artificiel permet de configurer facilement et de façon économique une salle opératoire 1 avec des dispositifs de suspension comme s'ils se trouvaient en position type 9-3 : un seul point d'ancrage est nécessaire et il y a suppression d'articulations tournantes.

Cet agencement avec déport artificiel présente tous les avantages d'une installation 9-3. Ces avantages connus sont une limitation les collisions, un déplacement des équipements 7, 7' dans des positions non atteignables avec des dispositifs de suspension de l'art antérieur centrés sur la table opératoire 4, limitant ainsi les singularités autour de la zone d'utilisation.

Ce déport artificiel des équipements 7, 7' dans une salle opératoire 1 est compatible avec des flux laminaires prévus initialement pour un ancrage centralisé.

Le dispositif de suspension selon l'invention permet de diminuer les efforts liés à la manipulation des équipements 7, 7' fixés au bras ressort 10, 10', comme cela est connu avec les dispositifs de suspension en position 9-3.

Il va de soi que la présente invention ne saurait être limitée à la description qui précède d'un de ses modes de réalisation, susceptibles de subir quelques modifications sans pour autant sortir du cadre de l'invention.

Par exemple, selon un autre mode de réalisation, les bras de déport 8, 8' en alignement mutuel de part et d'autre d'un point central de la platine d'ancrage 2 tels illustrés sur la figure 2 pourrait être remplacé par un bras de déport 8" unique fixé en son milieu par une liaison unique sur point central de la platine d'ancrage 2 comme représenté sur la figure 5. La fixation peut être du type fourreau/collier pour également permettre de monter, de démonter et de régler en hauteur le bras de déport 8" unique avant et après une opération médicale.

## Revendications

1. Dispositif de suspension permettant de suspendre des équipements (7,7') sous une structure support (3), comprenant une platine d'ancrage (2) destinée à être ancrée axialement (XX') à ladite structure support (3), deux bras de distribution (9,10,9',10') réglables en débattement destinés à porter lesdits équipements (7,7'), chaque bras de distribution (9,10,9',10') comprenant un bras de suspension (9,9') ayant une extrémité articulée à ladite platine d'ancrage par l'intermédiaire d'une articulation pivot (11,11') permettant une rotation dudit bras de suspension (9,9') dans un plan horizontal et un bras ressort (10,10') articulé à l'autre extrémité dudit bras de suspension (9,9') pour être inclinable verticalement par rapport audit bras de suspension (9,9'), l'autre extrémité dudit bras ressort (10,10') portant un équipement (7,7'), **caractérisé en ce que** chaque articulation pivot (11,11') d'un bras de suspension (9,9') est disposée à l'extrémité d'un bras de déport (8,8' ; 8") pour être déportée horizontalement d'une certaine distance de déport par rapport audit axe (XX') de ladite platine d'ancrage (2) qui est supérieure à la longueur dudit bras de suspension (9,9'), et **en ce qu'**il comprend en outre un moyen de liaison qui solidarise de manière fixe ledit bras de déport (8,8'; 8") audit axe (XX') de ladite platine d'ancrage (2) de telle manière qu'il reste fixe par rapport à ladite platine d'ancrage (2) pendant un pivotement dudit bras de distribution (9,10,9',10') dans ledit plan horizontal.

2. Dispositif de suspension selon la revendication 1, **caractérisé en ce qu'**il comprend deux bras de déport (8,8') qui sont en alignement mutuel de part et d'autre d'un point central de ladite platine d'ancrage (2).

3. Dispositif de suspension selon l'une des revendications précédentes, **caractérisé en ce que** ledit équipement (7,7') est un équipement médical.

## Patentansprüche

1. Aufhängvorrichtung, die das Aufhängen von Ausrüstungen (7, 7') unter einer Tragstruktur (3) ermöglicht, umfassend eine Ankerplatte (2), die dazu bestimmt ist, axial (XX') an der Stützstruktur (3) verankert zu werden, zwei Verteilarme (9, 10, 9', 10'), die durch Herausziehen einstellbar sind und dazu bestimmt sind, die Ausrüstungen (7, 7') zu tragen, wobei jeder Verteilarm (9, 10, 9', 10') einen Aufhängarm (9, 9') mit einem Ende, das mittels eines eine Rotation des Aufhängarms (9, 9') in einer horizontalen Ebene ermöglichenden Schwenkgelenks (11, 11') an die Ankerplatte angelenkt ist, und einen Federarm (10, 10'), der an dem anderen Ende des Aufhängarms (9, 9') angelenkt ist, um vertikal in Bezug auf den Aufhängarm (9, 9') neigbar zu sein, umfasst, wobei das andere Ende des Federarms (10, 10') eine Ausrüstung (7, 7') trägt, **dadurch gekennzeichnet,**
**dass** jedes Schwenkgelenk (11, 11') eines Aufhängarms (9, 9') an dem Ende eines Versatzarms (8, 8'; 8") angeordnet ist, um horizontal um eine bestimmte Versatzstrecke in Bezug auf die Achse (XX') der Ankerplatte (2) versetzt zu werden, welche größer ist als die Länge des Aufhängarms (9, 9'), und
**dass** sie ferner ein Verbindungsmittel umfasst, welches auf feste Weise den Versatzarm (8, 8'; 8") mit der Achse (XX') der Ankerplatte (2) derart befestigt, dass er während einer Verschwenkung des Verteilarms (9, 10, 9', 10') in der horizontalen Ebene fest in Bezug auf die Ankerplatte (2) verbleibt.

2. Aufhängvorrichtung nach Anspruch 1, **dadurch gekennzeichnet, dass** sie zwei Versatzarme (8, 8') umfasst, die jeweils in Bezug auf einen zentralen Punkt der Ankerplatte (2) gegenseitig ausgerichtet sind.

3. Aufhängvorrichtung nach einem der vorherigen Ansprüche, **dadurch gekennzeichnet, dass** die Ausrüstung (7, 7') eine medizinische Ausrüstung ist.

## Claims

1. A suspension device making it possible to suspend items of equipment (7, 7') under a support structure (3), said suspension device comprising an anchor bracket (2) designed to be anchored axially (XX') to said support structure (3), two distribution arms (9, 10, 9', 10') that are adjustable up and down and that are designed to carry said items of equipment (7, 7'), each distribution arm (9, 10, 9', 10') comprising a suspension arm (9, 9') having one end articulated to said anchor bracket via a pivot articulation (11, 11') making it possible for said suspension arm (9, 9') to move in rotation in a horizontal plane, and a spring arm (10, 10') articulated to the other end of said suspension arm (9, 9') so as to be inclinable vertically relative to said suspension arm (9, 9'), the other end of said spring arm (10, 10') carrying a respective item of equipment (7, 7'), said suspension device being **characterized in that** each pivot articulation (11, 11') of a suspension arm (9, 9') is disposed at the end of an offsetting arm (8, 8'; 8") so as to be offset horizontally by a certain offset distance relative to said axis (XX') of said anchor bracket (2), which length is greater than the length of said suspension arm (9, 9'), and **in that** it further comprises connection means that secure said offsetting arm (8, 8'; 8") in stationary manner relative to said axis (XX') of said anchor bracket (2) in such a manner that it remains stationary relative to said anchor bracket (2) while said distribution arm (9, 10, 9', 10') is pivoting in said horizontal plane.

2. A suspension device according to claim 1, **characterized in that** it comprises two offsetting arms (8, 8') that are in mutual alignment on either side of a central point of said anchor bracket (2).

3. A suspension device according to either preceding claim, **characterized in that** said equipment (7, 7') is medical equipment.
